# EUROPEAN PATENT APPLICATION

(11) **EP 4 556 482 A1**
(43) Date of publication of application: **21.05.2025**
(21) Application number: 23839751.7
(22) Date of filing: 07.04.2023
(51) Int. Cl.: C07H 3/02

(54) **METHOD FOR PRODUCING D-ALLULOSE CRYSTALS**

(30) Priority: 11.07.2022 KR 20220085142
(71) Applicant: Daesang Corporation, Seoul 03130 (KR)
(72) Inventor: LEE, Chang Seon, Seoul 07789 (KR); BAEK, Seon Hwa, Seoul 02850 (KR); JANG, Yeon Ock, Gapyeong-gun Gyeonggi-do 12411 (KR); CHO, Seung Woo, Seoul 07789 (KR); KANG, Jeong Won, Seoul 05828 (KR); SONG, Eun Bum, Seoul 07789 (KR)
(74) Representative: Kehl, Ascherl, Liebhoff & Ettmayr Patentanwälte Partnerschaft mbB
(86) International application number: PCT/KR2023/004706
(87) International publication number: WO 2024/014657

(57) **Abstract**

The present disclosure provides a method for producing D-allulose crystals, comprising the steps of adding D-allulose seed crystals to a D-allulose-containing stock solution, and then carrying out a crystallization reaction under a temperature gradient in which the temperature of the D-allulose-containing stock solution is reduced from an initial temperature Tᵢ to a final temperature T_{f}. In the method for producing D-allulose crystals according to the present disclosure, the temperature gradient includes a temperature condition corresponding to the supersaturation state of a metastable zone, and the crystallization reaction is carried out under a reduced pressure condition of 10~100 millibars (mb). Most D-allulose crystal particles produced by the method of the present disclosure have a cubic crystal structure, and the particle size distribution of the crystal particles is uniform, resulting in good flowability. In addition, when D-allulose crystals are produced by the method of the present disclosure, the crystallization yield can be remarkably improved.

## Description

### [Technical Field]

The present disclosure relates to a method for producing D-allulose crystals, and more particularly, to a method for producing D-allulose crystals having a cubic crystal structure and a uniform particle size distribution of crystal particles.

### [Background Art]

D-allulose is also called D-psicose as an epimer of carbon at position 3 of fructose. The D-allulose is a functional monosugar that has 70% sweetness compared to sugar (Oshima 2006), but only 0.3% energy to be applied as a low-calorie sweetener in diet foods (Matsuo et al. 2002). In addition, the D-allulose has a function of suppressing glucose absorption and blood sugar and may be applied to food and drink for diabetic patients, food and drink for self-discipline, and the like, and may suppress the accumulation of abdominal fat through inhibition of enzyme activity involved in lipid synthesis in the liver and thus may be used for various functional foods such as health foods, and the like (Matsuo et al. 2001; Iida et al. 2008; Hayashi et al. 2010; Hossain et al. 2011).

With the characteristics, the allulose is a good source capable of replacing sugar, but belongs to rare sugar, which is monosugar that rarely exists in nature, and thus there is required a method of efficiently producing allulose to be applied to the food industry. The most efficient method for producing allulose for industrialization is a method for converting fructose to allulose using D-allulose 3-epimerase. Since a reaction solution containing D-allulose produced by an enzymatic reaction is a low-purity product containing about 30% (w/w) of D-allulose solid content, in order to prepare D-psicose crystal particles with high purity of 98% (w/w) or more, it is required to prepare a high-purity allulose-containing stock solution using chromatography.

Generally, a crystallization method of sugars is largely classified into two types in which the first is a concentration crystallization method, and the second is a cooling crystallization method. Both the methods utilize a principle of inducing crystal growth within a metastable zone of a supersaturated state as the crystallization method of sugars. Generally, the crystallization method of sugars is carried out in the metastable zone, which means the range of the concentration of the solution from an equilibrium concentration, i.e., a saturation concentration, to the lowest supersaturation at which crystals are spontaneously precipitated. At the concentration in the metastable zone, a crystallization phenomenon such as crystal nucleation does not occur, but when new crystals are added from the outside, the crystal growth occurs and thus a crystal size is increased. That is, in order to generate crystals, when seed crystals are added to a solution at a saturated concentration or more, the seed crystals are grown in the metastable zone to make the crystal growth. If the crystallization solution is excessively concentrated or cooled rapidly, the crystallization solution becomes a supersaturated state exceeding the metastable zone, and new crystal nucleation occurs instead of the crystal growth, which hinders crystal growth due to an increase in the number of individuals, and thus a temperature condition and an initial supersaturation concentration are important for crystal growth.

The D-allulose has a characteristic which is almost not changed at a crystal generation rate and a crystal growth rate even in the supersaturated concentration range and thus may be classified as sugars in which a particle size growth crystallization condition is difficult. Generally, in the sugar crystallization industry, the particle size of the crystal particle is known as an important factor, and when crystals produced in a mass production system are microcrystals, the separation of the crystals and the stock solution in crystal centrifuge equipment is not easily carried out due to the viscosity in the supersaturated concentration range, and thus the purity of the final product is reduced due to the influence of the remaining stock solution. In addition, the remaining stock solution also causes aggregation between the crystals during drying, which reduces the final product packaging amount or reduces marketability. Accordingly, these microcrystals are not suitable for a mass-production method.

With respect to the method for producing D-allulose crystals, Korean Patent Registration No. 10-1189640 discloses a method for producing D-psicose crystals, including steps of purifying a D-psicose solution treated with a decolorizing agent and an ion exchange resin, obtaining a purified D-psicose solution, and concentrating the purified D-psicose solution; and adding D-psicose seed crystals to the concentrated D-psicose solution in an amount of 0.01 to 1% (g/g) of the total amount of D-psicose in the concentrated D-psicose solution to crystallize D-psicose in the supersaturated state of a metastable zone. In addition, Korean Patent Registration No. 10-1749527 discloses a method for producing D-psicose crystals, including steps of removing impurities from a D-psicose solution to obtain a purified D-psicose solution; concentrating the purified D-psicose solution to 80 to 85 Brix(%); cooling the concentrated D-psicose solution to 30°C to 40°C at a rate of 5°C to 20°C per hour through a heat exchanger; crystallizing the 30°C to 40°C D-psicose solution within a range of 30°C to 40°C to obtain a masket; and carrying out main crystallization at 30°C to 40°C using the sub-crystallized masket.

### [Disclosure]

### [Technical Problem]

The present disclosure is derived under the technical background of the related art, and an object of the present disclosure is to provide a method for producing allulose crystals having a cubic crystal structure and a uniform particle size distribution of crystal particles. In addition, another object of the present disclosure is to provide a method for producing D-allulose crystals with improved crystallization yield.

### [Technical Solution]

The present inventors have confirmed the problems that when adding seed crystals to a D-allulose-containing stock solution and inducing crystallization while cooling under a temperature gradient condition of a metastable zone at normal pressure, crystals having a long needle-shaped structure are generated, and the crystals grown during the crystallization process are broken and micronized, resulting in an ununiform particle size distribution, and when the allulose crystals generated after the crystallization reaction is completed and the remaining stock solution is separated by a method such as filtration, the filtration performance is significantly reduced due to the microcrystal particles. In order to solve these problems, the present inventors have confirmed that when inducing allulose crystals from a D-allulose-containing stock solution, it is possible to produce allulose crystals having a cubic crystal structure, a large average particle size of crystal particles, and a uniform particle size distribution by using a reduced pressure condition within a predetermined range, and then completed the present disclosure. In addition, the present inventors have confirmed that in order to suppress new crystal nucleation when inducing allulose crystals from a D-allulose-containing stock solution, when adding distilled water or a low-concentration stock solution in a predetermined amount from the time when microcrystals are formed to the time when a crystallization reaction is completed, the crystallization yield was increased significantly, and then completed the present disclosure.

As used herein, the term "supersaturation state" means an unstable state in which a solute is dissolved beyond the solubility capacity of a solvent, and a state in which the solute may be precipitated as a solid. Accordingly, the supersaturation state is necessarily required to separate the solute in a solution by crystallization. In general, the supersaturation state of the solution may be affected by external conditions, impurities, temperature, concentration, pH, etc.

As used herein, the term "supersaturation state of the metastable zone" means a state in which the concentration of the solution is in a range from the saturation concentration to the lowest supersaturation concentration at which crystals are spontaneously precipitated. At the concentration in the metastable zone, a crystallization phenomenon such as crystal nucleation does not occur, but due to the supersaturation concentration, when crystals are added from the outside, the crystal growth occurs spontaneously and thus a crystal size is increased. That is, in order to generate crystals, when seed crystals are added to a solution at a saturation concentration or more, the seed crystals are grown in the metastable zone to form large crystals.

As used herein, the term the supersaturation state of "an unstable zone" means a state in which the concentration of the solution is in a range exceeding the lowest supersaturation concentration. Spontaneous crystal nucleation occurs in the concentration of the unstable zone.

In this specification, Brix, which is a unit of solid concentration of the D-allulose-containing stock solution, may be used interchangeably with wt%.

In order to solve the object, the present disclosure provides a method for producing D-allulose crystals, including steps of adding D-allulose seed crystals to a D-allulose-containing stock solution, and then carrying out a crystallization reaction under a temperature gradient in which the temperature of the D-allulose-containing stock solution is reduced from an initial temperature Tᵢ to a final temperature T_{f}.

In the present disclosure, an initial solid concentration of the D-allulose-containing stock solution is preferably 70 to 86 Brix, and more preferably 75 to 85 Brix, when considering smooth crystallization of D-allulose, the size of D-allulose crystal particles, the economic feasibility of the crystallization reaction, and the like. In addition, an initial D-allulose content of the D-allulose-containing stock solution is preferably 94 to 99 wt%, and more preferably 95 to 98 wt%, based on the total weight of sugars in the stock solution, when considering the size of the D-allulose crystal particles, the purity of the D-allulose crystal particles, the economic feasibility of the crystallization reaction, and the like. For example, the sugar composition of the D-allulose-containing stock solution may be composed of 95 to 98 wt% of allulose, 0.1 to 2 wt% of fructose, 0.5 to 4 wt% of glucose, and the remainder of other sugars, based on the total weight of sugars in the stock solution. The D-allulose-containing stock solution used as a starting material in the present disclosure may be prepared by various known methods. For example, the D-allulose-containing stock solution may be prepared by reacting fructose syrup with D-allulose 3-epimerase to prepare an allulose-containing solution, and then subjecting the solution to decolorization, desalting, fractionation by chromatography, and concentration.

In the present disclosure, the D-allulose seed crystals are microcrystals composed of high-purity D-allulose, and the D-allulose purity of the D-allulose seed crystals is preferably 99 to 100% (w/w), and more preferably 99.5 to 100% (w/w). In addition, the average particle size of the D-allulose seed crystals is not particularly limited and may be selected from 50 to 300 µm, and preferably 100 to 250 µm. The amount of the D-allulose seed crystals added is not particularly limited, and considering the size of the D-allulose crystal particles, the economic feasibility of the crystallization reaction, etc., the amount of the D-allulose seed crystals added is preferably 0.1 to 5.0% (w/w), and more preferably 0.2 to 3.0% (w/w) of the total weight of the solid content of the D-allulose-containing stock solution.

In the present disclosure, the initial temperature Tᵢ for the crystallization reaction is preferably selected from 30 to 55°C, and more preferably selected from 35 to 52°C, considering smooth crystallization of D-allulose, the size of the D-allulose crystal particles, the economic feasibility of the crystallization reaction, etc. If the initial temperature Tᵢ is less than 30°C, there is a concern that a solidification phenomenon may occur during the crystallization reaction, which may lower the degree of crystallization (or crystallization yield), making it difficult to be applied in industrial applications. In addition, if the initial temperature Tᵢ exceeds 55°C, the solid concentration of the D-allulose-containing stock solution corresponding to the supersaturated state may be too large, so that processes such as stirring may not be smooth. In the present disclosure, the final temperature T_{f} for the crystallization reaction may be selected from temperatures 1 to 15°C lower than the initial temperature Tᵢ, and preferably selected from temperatures 1 to 10°C lower than the initial temperature Tᵢ. In particular, as described below, when the crystallization reaction is carried out under a reduced pressure condition close to a vacuum state of 10 to 100 millibars (mb), even if the final temperature T_{f} is selected from temperatures 1 to 5°C lower than the initial temperature Tᵢ, crystal particles having a cubic crystal structure and a uniform particle size distribution may mainly be obtained. In the present disclosure, the temperature gradient in which the temperature of the D-allulose-containing stock solution is reduced from the initial temperature Tᵢ to the final temperature T_{f} to induce the crystallization reaction includes at least a temperature condition corresponding to the supersaturated state of the metastable zone, and preferably consists of a temperature condition that is maintained in the supersaturated state of the metastable zone while the crystallization reaction is carried out.

In the present disclosure, the crystallization reaction time is not particularly limited, and considering the size of the D-allulose crystal particles, the economic feasibility of the crystallization reaction, etc., the crystallization reaction time is preferably selected from 35 to 100 hrs, and more preferably selected from 40 to 80 hrs.

In the present disclosure, the crystallization reaction is carried out under a reduced pressure condition of 10 to 100 millibars (mb), preferably 30 to 80 millibars (mb). When the crystallization reaction is carried out under a reduced pressure condition close to a vacuum state, the generated crystal particles mainly have a cubic crystal structure and the particle size distribution of the crystal particles becomes uniform, so that when the generated crystals are separated from the stock solution after the crystallization reaction is completed, filtration is smooth and the flowability of the obtained crystal particles is improved.

The method for producing the D-allulose crystals according to the present disclosure may preferably further include controlling the concentration of the D-allulose-containing stock solution by adding a diluted solution to the D-allulose-containing stock solution while the crystallization reaction is carried out. In addition, the addition of the diluted solution may be carried out by adding water alone, adding a diluted stock solution having a lower solid concentration than the D-allulose-containing stock solution alone, or alternately adding water and the D-allulose-containing solution. The solid concentration of the D-allulose-containing diluted stock solution used as the diluted solution is preferably 10 to 45 Brix in order to suppress a rapid change in the concentration of the D-allulose-containing stock solution while the crystallization reaction is carried out at the time of addition of the diluted solution. In addition, the sugars composition of the D-allulose-containing solution alternately added with the water is preferably the same as the sugars composition of the D-allulose-containing stock solution. In addition, the solid concentration of the D-allulose-containing solution alternately added with the water is preferably 55 to 80 Brix in order to suppress a rapid change in the concentration of the D-allulose-containing stock solution while the crystallization reaction is carried out at the time of addition. In the present disclosure, the reason for controlling the concentration of the D-allulose-containing stock solution by adding the diluted solution while the crystallization reaction is carried out is to suppress the formation of new microcrystals during the crystallization reaction or to remove the formed microcrystals. During the crystallization reaction, if the D-allulose-containing stock solution is in a supersaturated state in the unstable zone due to various factors such as reaction temperature, reaction pressure, crystal precipitation, etc., D-allulose microcrystals such as crystal nuclei are formed, and the microcrystals have a negative effect on the structure of crystal particles, the size of crystal particles, the particle size distribution of crystal particles, etc. In the present disclosure, the diluted solution is added to the D-allulose-containing stock solution at least once, preferably multiple times (for example, 2 to 4 times), from the time when new microcrystals are formed while the crystallization reaction is carried out to the time when the crystallization reaction is completed, thereby minimizing the time when the D-allulose-containing stock solution is in a supersaturated state in the unstable zone, maintaining most of the time in the supersaturated state in the metastable zone, and suppressing the formation of new microcrystals caused by various factors or removing the formed microcrystals. In the present disclosure, the time when the new microcrystals are formed while the crystallization reaction is carried out is determined by various factors, such as a D-allulose-containing stock solution condition, a temperature gradient condition, a stirring condition, a pressure condition, etc., and is generally about 20 to 24 hrs after the D-allulose seed crystals are added to the D-allulose-containing stock solution and the crystallization reaction is carried out under a temperature gradient. In addition, in the present disclosure, the diluted solution is added while the crystallization reaction is carried out to control the structure of the crystal particles and the particle size distribution of the crystal particles and significantly increase the precipitation rate of the crystal particles. In the method for producing the D-allulose crystals according to the present disclosure, the total added amount of the diluted solution is not particularly limited, and considering an efficient concentration control of the D-allulose-containing stock solution, smooth suppression or removal of microcrystal formation, etc., the amount of the diluted solution is preferably 1 to 10% (w/w), and more preferably 2 to 8% (w/w) with respect to the total weight of the D-allulose-containing stock solution.

The method for producing the D-allulose crystals according to the present disclosure may preferably further include washing and dehydrating the stock solution after the crystallization reaction is completed, and drying the stock solution to obtain D-allulose crystal particles. The process of washing and dehydrating the stock solution may be implemented by various known methods, such as centrifugation, reduced pressure filtration using filter paper or filter cloth, etc. In addition, the drying process may be implemented by various known methods, such as hot air drying, spray drying, and fluidized bed drying.

Most of the D-allulose crystal particles produced by the method for producing the D-allulose crystals according to the present disclosure have a cubic crystal structure, and a relatively uniform particle size distribution, resulting in excellent flowability. In addition, the average particle size of the D-allulose crystal particles produced by the method for producing the D-allulose crystals according to the present disclosure is 200 to 500 µm, preferably 220 to 460 µm. In addition, the D-allulose crystal particles produced by the method for producing the D-allulose crystals according to the present disclosure have the D-allulose purity of 98.5% (w/w) or more (e.g., 98.5 to 100%), preferably 99% (w/w) or more (e.g., 99 to 99.9%).

### [Advantageous Effects]

According to the present disclosure, most D-allulose crystal particles produced by the method of the present disclosure have a cubic crystal structure, and the particle size distribution of the crystal particles is uniform, resulting in good flowability. In addition, when D-allulose crystals are produced by the method of the present disclosure, the crystallization yield can be remarkably improved.

### [Description of Drawings]

FIG. 1 shows a saturation curve and a supersaturation curve of D-allulose crystals.
FIG. 2 is a photograph captured under a microscope of D-allulose crystals obtained in Preparation Example 6 of the present disclosure.
FIG. 3 is a photograph captured under a microscope of D-allulose crystals obtained in Preparation Example 13 of the present disclosure.

### [Modes of the Invention]

Hereinafter, the present disclosure will be described in detail through the following Examples. However, the following Examples are only for clearly illustrating the technical features of the present disclosure, but do not limit the protection scope of the present disclosure.

### Example 1: Preparation of D-allulose-containing stock solution

An allulose epimerase variant derived from *Flavonifractor plautii* was added to a fructose-containing solution and reacted, and then centrifuged to recover a supernatant. With respect to a process of converting fructose into allulose using the allulose epimerase variant, the present disclosure refers to Korean Patent Publication No. 10-2021-0132405 and Korean Patent Registration No. 10-2254411. Thereafter, the supernatant was decolorized by treatment with activated carbon, desalted by passing through an ion exchange resin column, and purified to obtain a low-purity allulose-containing solution. Thereafter, the low-purity allulose-containing solution was concentrated to a solid concentration of about 50 Brix, and the low-purity allulose-containing concentrate passed through a calcium group-substituted ion exchange resin (MUK 555) to perform chromatographic separation, thereby obtaining a high-purity D-allulose-containing solution. Thereafter, the high-purity D-allulose-containing solution was concentrated to prepare a high-purity D-allulose-containing stock solution having various solid concentrations. The solid concentration of the D-allulose-containing stock solution was 70 to 84 Brix, and the sugar composition included 95.6% (w/w) of allulose, 1.0% (w/w) of fructose, 1.9% (w/w) of glucose, and 1.5% (w/w) of others based on the total solid weight.

### Example 2: Preparation of D-allulose seed crystals

D-allulose seed crystals were prepared using a D-allulose-containing stock solution having a solid concentration of 84 Brix. Specifically, the stock solution was placed in a crystallizer equipped with a stirring and temperature control system, the temperature of the stock solution was controlled to 40°C, and then 95% or more of a reagent-grade allulose particle product was added thereto in an amount of about 3 wt% based on the solid content of the stock solution, and stirred at a speed of about 100 rpm to evenly disperse the reagent-grade allulose particle product. Thereafter, the stirring speed was reset to about 1.0 to 1.5 rpm, and the crystallization reaction was carried out under temperature gradient conditions in which the temperature of the stock solution was slowly cooled from 40°C to 10°C for about 70 hrs. Thereafter, the solution in which the crystallization reaction had been carried out was washed and dehydrated using centrifugation, and dried at about 50°C for 6 hrs to obtain D-allulose seed crystals. The obtained D-allulose seed crystals had an average crystal particle size of about 150 µm, and the sugar composition was 99.9 wt% allulose content.

### Example 3: Measurement of saturation concentration and supersaturation concentration of D-allulose crystals

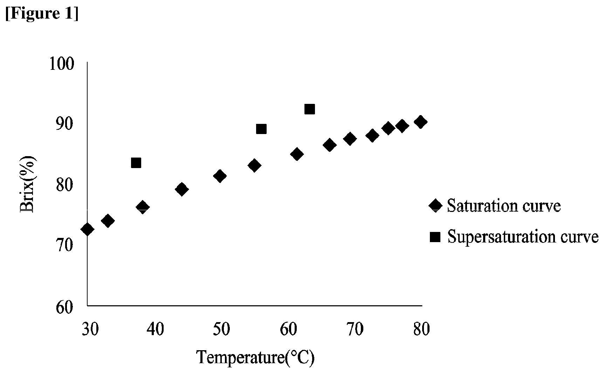

Under a specific temperature condition selected from 30 to 80°C, the D-allulose seed crystals obtained in Example 2 were dissolved in distilled water in small amounts, and a concentration at which the D-allulose seed crystals were no longer dissolved was defined as a saturation concentration at the corresponding temperature. In addition, high-purity D-allulose solutions having solid concentrations of 83 Brix, 89 Brix, and 92 Brix were prepared using the D-allulose seed crystals obtained in Example 2, respectively, and then a temperature at the time when crystal particles began to be formed was measured while slowly cooling, and the concentration of each sample was defined as a supersaturation concentration at the corresponding temperature. FIG. 1 shows a saturation curve and a supersaturation curve of D-allulose crystals.

### Example 4: Crystallization experiment according to normal pressure condition and temperature gradient in metastable zone of D-allulose-containing stock solution

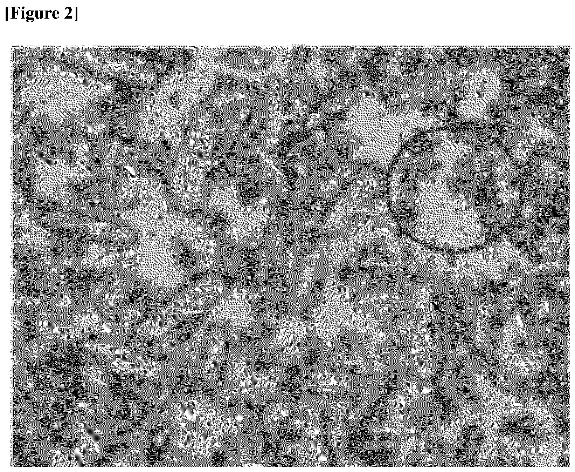

Allulose crystals were prepared using a D-allulose-containing stock solution of various solid concentrations. Specifically, the stock solution was placed in a crystallizer equipped with a stirring and temperature control system, and the temperature of the stock solution was controlled to a specific temperature. Then, the D-allulose seed crystals obtained in Example 2 were added thereto in a predetermined amount compared to the solid content of the stock solution, and stirred at a speed of about 100 rpm to evenly disperse the D-allulose seed crystals. Thereafter, the stirring speed was reset to about 1.0 to 1.5 rpm, and the stock solution was cooled under a normal pressure condition over a predetermined period of time or maintained at the cooled temperature for a predetermined period of time to carry out the crystallization reaction under the temperature gradient condition corresponding to the metastable zone. Thereafter, the solution in which the crystallization reaction had been carried out was washed and dehydrated using centrifugation, and dried at about 50°C for 6 hrs to obtain D-allulose crystals. The crystallization reaction condition used in Example 4 and the analysis result of the obtained D-allulose crystals were shown in Tables 1 and 2 below. FIG. 2 is a photograph captured under a microscope of D-allulose crystals obtained in Preparation Example 6 of the present disclosure. As shown in FIG. 2, when seed crystals were added to the allulose-containing stock solution and crystallization was induced while cooling under a temperature gradient condition of the metastable zone at normal pressure, crystals having a long needle-shaped structure were generated, and the crystals grown during the crystallization process were broken and micronized, resulting in an ununiform particle size distribution. When the solution in which the crystallization reaction was completed in Preparation Example 6 was filtered by centrifugation or the like, a dense film was formed on the filter cloth due to the micronized crystal particles, and the filtration performance was significantly reduced. In addition, the D-allulose crystal particles prepared in Preparation Example 6 had a large number of microcrystal particles and a particle size distribution was ununiform, resulting in poor flowability even after drying.

**[Table 1]**

| Item classification | | Preparation Example 1 | Preparation Example 2 | Preparation Example 3 |
|---|---|---|---|---|
| Crystalliz ation reaction condition | Stock solution solid concentration (Brix) | 80.4 | 80.3 | 80.3 |
| | Added amount of seed crystals (wt% compared to stock solution solid content) | 2.0 | 0.3 | 0.3 |
| | Pressure (atm) | 1 | 1 | 1 |
| | Temperature gradient (°C) | 46.5→44.5 | 38.0→32.0 | 38.0→37.0 |
| | Reaction time (hr) | 72 | 72 | 72 |
| Crystal particle analysis result | Average particle size (µµm) | 195.0 | 147.5 | 250.0 |
| | Particle size standard deviation (µm) | 115.0 | 125.0 | 110.0 |
| | Precipitation rate (%) | 21.2 | 17.3 | 29.0 |
| | Crystal particle shape | Mainly long needle shape | Mainly long needle shape | Mainly long needle shape |

**[Table 2]**

| Item classification | | Preparation Example 4 | Preparation Example 5 | Preparation Example 6 |
|---|---|---|---|---|
| Crystalliz ation reaction condition | Stock solution solid concentration (Brix) | 82.3 | 80.7 | 80.8 |
| | Added amount of seed crystals (wt% compared to stock solution solid content) | 0.3 | 1.0 | 0.3 |
| | Pressure (atm) | 1 | 1 | 1 |
| | Temperature gradient (°C) | 48.5→39.5 | 42.0 | 45.0→34.0 |
| | Reaction time (hr) | 72 | 72 | 72 |
| Crystal particle analysis result | Average particle size (µm) | 305.0 | 300.0 | 185.0 |
| | Particle size standard deviation (µm) | 150.0 | 140.0 | 120.0 |
| | Precipitation rate (%) | 48.2 | 46.2 | 38.7 |
| | Crystal particle shape | Mainly long needle shape | Mainly long needle shape | Mainly long needle shape |

### Example 5: Preliminary experiment for setting pressure condition in crystallization process according to temperature gradient in metastable zone of D-allulose-containing stock solution

A D-allulose-containing stock solution having a solid concentration of 81 Brix was placed in a crystallizer equipped with a stirring, temperature control, and pressure control system, and the temperature of the stock solution was controlled to 45°C. Then, the D-allulose seed crystals obtained in Example 2 were added thereto in an amount of 0.5 wt% based on the solid content of the stock solution, and stirred at a speed of about 100 rpm to evenly disperse the D-allulose seed crystals. Thereafter, the stirring speed was reset to about 1.0 to 1.5 rpm, and the internal pressure of the crystallizer was gradually reduced from 250 millibars (mb) to 50 millibars (mb) to observe crystal formation in the allulose-containing stock solution. When the growth of D-allulose crystals began under a near-vacuum condition with an internal pressure of the crystallizer of 100 millibars (mb) or less, a whitening phenomenon appeared.

### Example 6: Crystallization experiment according to reduced pressure condition and temperature gradient in metastable zone of D-allulose-containing stock solution

Allulose crystals were prepared using a D-allulose-containing stock solution having various solid concentrations. Specifically, the stock solution was placed in a crystallizer equipped with a stirring, temperature control, and pressure control system, and the temperature of the stock solution was controlled to a specific temperature. Then, the D-allulose seed crystals obtained in Example 2 were added thereto in a predetermined amount compared to the solid content of the stock solution, and stirred at a speed of about 100 rpm to evenly disperse the D-allulose seed crystals. Thereafter, the stirring speed was reset to about 1.0 to 1.5 rpm, and the stock solution was cooled over a predetermined period of time while controlling the reduced pressure conditions to a predetermined range or maintained at the cooled temperature for a predetermined period of time to carry out the crystallization reaction under the temperature gradient condition corresponding to the metastable zone. Thereafter, the solution in which the crystallization reaction had been carried out was washed and dehydrated using centrifugation, and dried at about 50°C for 6 hrs to obtain D-allulose crystals. The crystallization reaction condition used in Example 6 and the analysis result of the obtained D-allulose crystals were shown in Tables 3 and 4 below.

**[Table 3]**

| Item classification | | Preparation Example 7 | Preparation Example 8 | Preparation Example 9 |
|---|---|---|---|---|
| Crystalliz ation reaction condition | Stock solution solid concentration (Brix) | 82.6 | 81.1 | 82.4 |
| | Added amount of seed crystals (wt% compared to stock solution solid content) | 0.3 | 1.0 | 1.0 |
| | Pressure (millibar, mb) | 50~60 | 50~60 | 50~60 |
| | Temperature gradient (°C) | 48.0→47.0 | 44.0→43.0 | 48.0→47.0 |
| | Reaction time (hr) | 48 | 48 | 48 |
| Crystal particle analysis result | Average particle size (µm) | 455.0 | 445.0 | 335.0 |
| | Particle size standard deviation (µm) | 75.0 | 95 | 60.0 |
| | Precipitation rate (%) | 6.6 | 10.3 | 14.0 |
| | Crystal particle shape | Mainly cubic shape | Mainly cubic shape | Mainly cubic shape |

**[Table 4]**

| Item classification | | Preparation Example 10 | Preparation Example 11 | Preparation Example 12 |
|---|---|---|---|---|
| Crystalliz ation reaction condition | Stock solution solid concentration (Brix) | 80.7 | 80.8 | 80.4 |
| | Added amount of seed crystals (wt% compared to stock solution solid content) | 1.0 | 3.0 | 2.0 |
| | Pressure (millibar, mb) | 50~60 | 50~60 | 50~60 |
| | Temperature gradient (°C) | 46.0→45.0 | 46.0→45.0 | 46.0→45.0 |
| | Reaction time (hr) | 72 | 72 | 72 |
| Crystal particle analysis result | Average particle size (µm) | 325.0 | 275.0 | 275.0 |
| | Particle size standard deviation (µm) | 80.0 | 92.5 | 80.0 |
| | Precipitation rate (%) | 16.7 | 18.2 | 28.4 |
| | Crystal particle shape | Mainly cubic shape | Mainly cubic shape | Mainly cubic shape |

### Example 7: Crystallization experiment according to reduced pressure condition, temperature gradient in metastable zone, and addition of diluted solution of D-allulose-containing stock solution

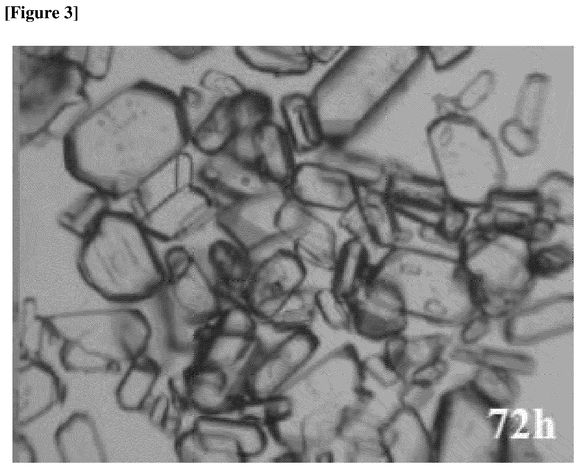

Allulose crystals were prepared using a D-allulose-containing stock solution having various solid concentrations. Specifically, the stock solution was placed in a crystallizer equipped with a stirring, temperature control, and pressure control system, and the temperature of the stock solution was controlled to a specific temperature. Then, the D-allulose seed crystals obtained in Example 2 were added thereto in a predetermined amount compared to the solid content of the stock solution, and stirred at a speed of about 100 rpm to evenly disperse the D-allulose seed crystals. Thereafter, the stirring speed was reset to about 1.0 to 1.5 rpm, and the stock solution was cooled over a predetermined period of time while controlling the reduced pressure condition to a predetermined range or maintained at the cooled temperature for a predetermined period of time to carry out the crystallization reaction under the temperature gradient condition corresponding to the metastable zone. In addition, from the time when microcrystals were generated while the crystallization reaction was carried out (when the crystallization reaction time was about 24 hrs) to the time when the crystallization reaction was completed, as a dilute solution, distilled water alone, a D-allulose-containing diluted stock solution having a solid content of 35 Brix alone, or a combination of distilled water and a D-allulose-containing stock solution having a solid content of 70 Brix were added total twice at regular intervals to control the concentration of the stock solution and suppress new crystal nucleation. The total amount of the diluted solution added was 5% (w/w) based on the total weight of the D-allulose-containing stock solution used for the crystallization reaction. Thereafter, the solution in which the crystallization reaction had been carried out was washed and dehydrated using centrifugation, and dried at about 50°C for 6 hrs to obtain D-allulose crystals. The crystallization reaction condition used in Example 7 and the analysis result of the obtained D-allulose crystals were shown in Table 5 below. FIG. 3 is a photograph captured under a microscope of D-allulose crystals obtained in Preparation Example 13 of the present disclosure. As shown in FIG. 3, when seed crystals were added to the allulose-containing stock solution and the crystallization was induced while cooling under a temperature gradient condition of a metastable zone under a reduced pressure condition, and a diluted solution was added while the crystallization reaction was carried out to control the concentration of the stock solution and suppress the formation of new microcrystals, crystals having a cubic crystal structure were formed, and a phenomenon in which the crystals growing during the crystallization process were broken was minimized, so that the particle size distribution was uniform. When the solution in which the crystallization reaction was completed in Preparation Example 13 was filtered through centrifugation, etc., there was almost no decrease in filtration performance. In addition, the D-allulose crystal particles prepared in Preparation Example 13 had an average particle size within an appropriate range and a particle size distribution was uniform, resulting in good flowability even after drying.

**[Table 5]**

| Item classification | | Preparation Example 10 | Preparation Example 11 | Preparation Example 12 |
|---|---|---|---|---|
| Crystalliz ation reaction condition | Stock solution solid concentration (Brix) | 81.9 | 80.1 | 80.5 |
| | Added amount of seed crystals (wt% compared to stock solution solid content) | 0.3 | 0.3 | 0.25 |
| | Pressure (millibar, mb) | 50~70 | 50~70 | 50~70 |
| | Temperature gradient (°C) | 48.6→46.0 | 47.0→45.0 | 51.5→49.5 |
| | Diluted solution addition condition | Addition of distilled water alone | Addition of D-allulose-containing diluted stock solution with solid content of 35 Brix | Alternate addition of distilled water and D-allulose-containing stock solution with solid content of 70 Brix |
| | Reaction time (hr) | 72 | 72 | 72 |
| Crystal particle analysis result | Average particle size (µm) | 245.0 | 195.0 | 220.0 |
| | Particle size standard deviation (µm) | 85.0 | 75.0 | 80.0 |
| | Precipitation rate (%) | 90.4 | 74.1 | 99.0 |
| | Crystal particle shape | Mainly cubic shape | Mainly cubic shape | Mainly cubic shape |

As described above, the present disclosure has been described through Examples above, but the present disclosure is not necessarily limited thereto, and various modifications may be made without departing from the scope and spirit of the present disclosure. Therefore, the scope of the present disclosure should be construed to include all embodiments falling within the scope of claims appended hereto.

## Claims

1. A method for producing D-allulose crystals, comprising adding D-allulose seed crystals to a D-allulose-containing stock solution, and then carrying out a crystallization reaction under a temperature gradient in which the temperature of the D-allulose-containing stock solution is reduced from an initial temperature Tᵢ to a final temperature T_{f},
wherein the temperature gradient includes a temperature condition corresponding to the supersaturation state of a metastable zone, and
the crystallization reaction is carried out under a reduced pressure condition of 10 to 100 millibars (mb).

2. The method for producing D-allulose crystals of claim 1, further comprising:
controlling the concentration of the D-allulose-containing stock solution by adding a diluted solution to the D-allulose-containing stock solution while the crystallization reaction is carried out.

3. The method for producing D-allulose crystals of claim 1, wherein an initial solid concentration of the D-allulose-containing stock solution is 70 to 86 Brix, and an initial D-allulose content of the D-allulose-containing stock solution is 94 to 99 wt% based on the total weight of sugars in the stock solution.

4. The method for producing D-allulose crystals of claim 1, wherein the added amount of the D-allulose seed crystals is 0.1 to 5.0% (w/w) based on the total weight of the solid content of the D-allulose-containing stock solution.

5. The method for producing D-allulose crystals of claim 1, wherein the initial temperature Tᵢ is selected from 30 to 55°C, and the final temperature T_{f} is selected from a temperature 1 to 15°C lower than the initial temperature Tᵢ.

6. The method for producing D-allulose crystals of claim 1, wherein the crystallization reaction time is selected from 35 to 100 hrs.

7. The method for producing D-allulose crystals of claim 2, wherein the addition of the diluted solution is carried out by adding water alone, adding a diluted stock solution having a lower solid concentration than the D-allulose-containing stock solution alone, or alternately adding water and the D-allulose-containing solution.

8. The method for producing D-allulose crystals of claim 2, wherein the addition of the diluted solution is carried out multiple times from the time when microcrystals are formed to the time when the crystallization reaction is completed.

9. The method for producing D-allulose crystals of claim 2, wherein the total amount of the diluted solution added is 1 to 10% (w/w) based to the total weight of the D-allulose-containing stock solution.
